# NEUE EUROPÄISCHE PATENTSCHRIFT

(11) **EP 1 948 131 B3**
(45) Veröffentlichungstag dieser Patentschrift: **27.03.2013**
(45) Hinweis auf die Patenterteilung: 18.03.2009
(21) Anmeldenummer: 06818627.9
(22) Anmeldetag: 17.11.2006
(51) Int. Cl.: A61K 9/08, A61K 31/194, A61K 47/36, A61P 27/02

(54) **DEXPANTHENOL-, CALCIUMIONEN- UND PHOSPHATFREIE PHARMAZEUTISCHE ZUSAMMENSETZUNG SOWIE VERWENDUNG VON CALCIUM-CHELATBILDNER UND OPHTHALMOLOGISCH VERTRÄGLICHEM VISKOSITÄTSREGLER**
PHARMACEUTICAL COMPOSITION FREE FROM DEXPANTHENOL, CALCIUM IONS, AND PHOSPHATE, AND USE OF CALCIUM CHELATING AGENT AND OPHTHALMOLOGICALLY COMPATIBLE VISCOSITY REGULATOR
COMPOSITION PHARMACEUTIQUE EXEMPTE DE DEXPANTHENOL, D'IONS DE CALCIUM ET DE PHOSPHATE, UTILISATION DE CHELATEURS DU CALCIUM ET D'UN REGULATEUR DE VISCOSITE ACCEPTABLE SUR LE PLAN OPHTALMIQUE

(30) Priorität: 17.11.2005 DE 102005055275
(43) Veröffentlichungstag der Anmeldung: 30.07.2008
(73) Patentinhaber: URSAPHARM Arzneimittel GmbH & Co. KG, 66129 Saarbrücken (DE)
(72) Erfinder: HOLZER, Frank, 66386 St. Ingbert (DE); GROSS, Dorothea, 66386 St. Ingbert (DE)
(74) Vertreter: Pfenning, Meinig & Partner GbR
(86) Internationale Anmeldenummer: PCT/EP2006/011053
(87) Internationale Veröffentlichungsnummer: WO 2007/057201

(56) Entgegenhaltungen:
- EP-A2- 0 464 727
- GB-A- 2 160 097
- JP-A- 2 096 515

## Beschreibung

Die Erfindung betrifft die Verwendung von einem Calcium-Chelatbildner und einem ophthalmologisch verträglichen Viskositätsregler zur Herstellung einer phosphatfreien pharmazeutischen Zusammensetzung zur Behandlung und/oder Prävention von Epitheldefekten.

Viskositätsregler, insbesondere Hyaluronsäure bzw. Hyaluronate werden bei Benetzungsstörungen des Auges, d.h. bei dem Erkrankungsbild des "trockenen Auges", das auch als Sicca-Syndrom oder auch als Sicca-Symptomatik bezeichnet wird, und zur Behandlung von Epithelläsionen, die aus den Benetzungsstörungen resultieren, verwendet.

Bei dem Krankheitsbild des "trockenen Auges" äußern sich die Symptome unter anderem in Brennen, Kratzen, Sandkomgefühl im Auge und verschwommenem Sehen. Diese Symptome sind auf funktionelle Störungen des Tränenflusses bzw. des Tränenfilms zurückzuführen.

Die Verwendung von Phosphatpuffer- und Hyaluronsäure-haltigen Tropfen zur Therapie des Krankheitsbildes des Trockenen Auges ist bekannt (Israeli Journal of Medical Science 1997, 33, Seiten 194 bis 197, British Journal of Ophthalmology 2002, 86, Seiten 181-184). Ferner beschreibt die EP 0698388 A1 eine künstliche Tränenflüssigkeft, welche Hyaluronsäure, Calcium-Ionen. Citrat und Phosphat-Ionen umfasst.

Phosphate bilden jedoch mit dem körpereigenen Calcium oder den körpereigenen Calciumionen bzw. mit den in den pharmazeutischen Präparaten enthaltenen Calciumionen nachteiligerweise schwerlösliche Calciumphosphate, die sich in oder auf der Hornhaut sowie der Bindehaut des Auges einlagern oder ablagern können. Diese Ein- und/oder Ablagerungen können auch als Kalzifikation oder Verkalkung bezeichnet werden und führen zu einer erheblichen Beeinträchtigung des Sehvermögens durch Trübung der Hornhaut. Man bezeichnet diese Degeneration der Hornhaut auch als Homhautbanddegeneration oder bandförmige Keratopathie. Bereits geringfügige Trübungen der Hornhaut führen zu einer massiv erhöhten Blendempfindlichkeit, die auf eine an den Ab- oder Einlagerungen von Calciumphosphat(en) erfolgende Lichtstreuung zurückzuführen ist. Das Sehvermögen in der Nacht wird hierdurch stark beeinträchtigt. Insbesondere an Epitheldefekten der Hornhaut und/oder der Bindehaut kann es zur Ablagerung derartiger schwerlöslicher Calciumphosphate oder anderer schwerlöslicher Calciumverbindungen kommen.

Im Hinblick auf die möglichen Nebenwirkungen durch Calciumphosphatablagerungen wäre es mithin wünschenswert, über eine pharmazeutische Zusammensetzung zu verfügen, die die oben genannten Nachteile vermindert und/oder beseitigt und zur topischen Applikation am Auge geeignet ist.

Die DE 101 61 110 A1 offenbart eine pharmazeutische Zusammensetzung, die wenigstens Panthenol und/oder Panthotensäure und Hyaluronsäure und/oder Hyaluronat sowie optional pharmazeutische Hilfsmittel enthält. Die in der DE 101 61 110 A1 offenbarte pharmazeutische Zusammensetzung wird zur Behandlung des Krankheitsbildes des Trockenen Auges verwendet.

Die DE 602 03 691 T2 offenbart eine Dexpanthenol-haltige Kontaktlinsenpflegezusammensetzung. Die mit der Dexpanthenol-haltigen Pflegezusammensetzung behandelten Kontaktlinsen eignen sich zum Tragen im Falle von trokkenen und/oder gereizten Augen.

Die EP 0 414 373 A2 offenbart eine Calcium-haltige sowie phosphatfreie Hyaluronatsalz-haltige Zusammensetzung zur Verwendung als isotonische und osmotisch ausgeglichene Salzlösung während chirurgischer Operationen am Auge. Die EP 0 464 727 A2 betrifft eine Zusammensetzung zur Entfernung von zurückgehaltenem Augenlinsenmaterial während der Absaugung bei der Cataract-Chirurgie.

Die US 4,409,205 offenbart eine ophthalmische Lösung zur Verwendung bei der Normalisierung eines irregulär strukturierten Tränenfilmes in Säugetieraugen. Die aus der US 4,409,205 bekannte Zusammensetzung verhindert die Präzipitation von Protein-ähnlichen Substanzen aus den Tränen und fördert die Resolubilisierung von ausgefallenen Protein-ähnlichen Substanzen.

Die WO 84/04681 offenbart eine weitere ophthalmische Lösung zur Linderung der Beschwerden des trockenen Auges, wobei die Zusammensetzung ein Carboxyvinylpolymer enthält.

Die der Erfindung zugrundeliegende Aufgabe wird durch die Verwendung von mindestens einem Calcium-Chelatbildner und mindestens einem ophthalmologisch verträglichen Viskositätsregler zur Herstellung einer phosphatfreien pharmazeutischen Zusammensetzung zur Behandlung und/oder Prävention von Epitheldefekten in der Hornhaut und/oder Bindehaut des Auges gelöst.

Bevorzugte Weiterbildungen der erfindungsgemäßen Verwendung sind in den Unteransprüchen 2 bis 19 angegeben.

Die pharmazeutische Zusammensetzung wird topisch auf die Augenoberfläche, vorzugsweise eine Augenoberfläche mit Epitheldefekten in der Hornhaut und/oder Bindehaut des Auges, aufgebracht. Die Epitheldefekte können bspw. durch Verletzung und/oder Operationen am Auge verursacht sein.

Unter einer "phosphatfreien pharmazeutischen Zusammensetzung" wird im Sinne der Erfindung eine pharmazeutische Zusammensetzung verstanden, die weniger als 7 mmol/l Phosphationen, vorzugsweise weniger als 3 mmol/l Phosphationen, besonders bevorzugt weniger als 1 mmol/l Phosphationen und äußerst bevorzugt keine Phosphationen enthält.

Unter dem Begriff "Phosphationen" im Sinne der Erfindung werden insbesondere PO₄³⁻, HPO₄²⁻ und/oder H₂PO₄⁻ verstanden.

Als Viskositätsregler im Sinne der Erfindung werden Stoffe bezeichnet, die eine Viskositäts-erhöhende Wirkung haben.

Im Sinne der Erfindung wird unter"ophthalmologischverträglich"insbesondere verstanden, daß keine Reizungen des Auges und vorzugsweise keine Beeinträchtigungen des Sehvermögens auftreten.

Vorzugsweise weist der Viskositätsregler ein viskoelastisches Verhalten auf. Unter einem viskoelastischen Verhalten wird erfindungsgemäß verstanden, daß sich die Viskosität unter der Einwirkung von Druck-, Zug-, Schub-, und/oder, Scherspannungen ändert. Besonderes bevorzugt weist die erfindungsgemäß zu verwendende, phosphatfreie pharmazeutische Zusammensetzung aufgrund des Viskositätsreglers das Verhaften einer Nicht-Newtonschen-Flüssigkeit auf.

Die Viskosität liegt vorzugsweise in einem Bereich von 2 bis 1000 mPa•s, weiter bevorzugt in einem Bereich von 2 bis 500 mPa•s, besonders bevorzugt in eine Bereich von 2 bis 100 mPa•s.

Die Viskositäts-erhöhende Wirkung bewirkt äußerst vorteilhaft, daß die auf die Augenoberfläche aufgebrachte phosphatfreie pharmazeutische Zusammensetzung eine erhöhte Verweildauer aufweist und verlangsamt von der Augenoberfläche, wieder abfließt. Das Nicht-Newtonsche Fließverhalten des Viskositätsreglers bedingt eine für die Anwendung am Auge hervorragende Eigenschaft, dass nämlich die Viskosität mit zunehmender Schergeschwindigkeit abnimmt. Nach Aufbringung der phosphatfreien pharmazeutischen Zusammensetzung mit dem Viskositätsregler auf die Oberfläche des Auges wird über den Lidschlag des Augenlides eine Scherspannung an die phosphatfreie pharmazeutische Zusammensetzung angelegt, wodurch die zunächst erhöhte Viskosität erniedrigt wird. Durch den Lidschlag des Augenlides erniedrigt sich die Viskosität, so daß sich ein gleichmäßiger Film auf der Oberfläche des Auges ausbildet. Nach dem Lidschlag erhöht sich die Viskosität, so daß der Film an der Augenobenfläche gut anhaftet und nurverlangsamt abläuft.

Vorzugsweise wirkt der Viskositätsregler als Gleit- und Schmiermittel auf dem Auge. Die Gleit- und Schmierwirkung ist insbesondere dann vorteilhaft, wenn die Augenoberfläche, insbesondere die Hornhaut, Verletzungen, insbesondere Epithelläsionen, aufweist.

Gemäß einer bevorzugten Ausführungsform beträgt die Menge an Viskositätsregler etwa 0,005 Gew.-% bis etwa 5 Ges.-%, bevorzugt etwa 0,01 Gew.-% bis etwa 1 Gew.-%, jeweils bezogen auf das Gesamtgewicht der phosphatfreien pharmazeutischen Zusammensetzung.

Die erfindungsgemäß zu verwendende phosphatfreie pharmazeutische Zusammensetzung enthält als ophthalmologisch verträglichen Viskositätsreglervorzugsweise Chondroitinsulfat, Polyacrylamid, Polyacrylsäure, Polyacrylharze, Polyethylenglycol, Cellulosederivate, Polysaccharide, Polyvinylpyrrolidon, Hyaluronsäure, Hyaluronate, Derivate davon und/oder Mischungen davon. Besonders bevorzugt werden als ophthalmologisch verträgliche Viskositätsregler Hyaluronsäure, Hyaluronate, Derivate davon und/oder Mischungen davon verwendet,

Hyaluronsäure ist Bestandteil des Glaskörpers des Auges und stellt insofern keine für den menschlichen Organismus fremde Verbindung dar. Aus diesem Grund ist Hyaluronsäure aus immunologischer Sicht sehr gut verträglich. Darüber hinaus weist Hyaluronsäure bzw. Hyaluronat eine stru kturelle Ähnlichkeit mit Mucin auf. Mucin bildet die unterste Schicht des dreischichtigen Tränenfilms und sorgt für eine optimale Benetzung der Hornhaut- und Bindehautepithelien.

Weiterhin weist Hyaluronsäure eine für die Anwendung am Auge hervorragende Eigenschaft auf, dass nämlich die Viskosität mit Zunehmen der Schwergeschwindigkeit abnimmt. Die Hyaluronsäure weist somit ein Nicht-Newtonsches Fließverhalten auf.

Hyaluronsäure bzw. deren Salze, die Hyaluronate bzw. insbesondere Natrium-Hyaluronat weist bzw. weisen hervorragende optische Eigenschaften auf, so daß es zu keiner Beeinträchtigung des Sehvermögens bei den behandelten Patienten kommt.

Die Hyaluronsäure bzw. Hyaluronat kann aus dem Glaskörper des Rinderauges oder aber auch aus Hahnenkämmen isoliert werden. Weiterhin kann Hyaluronsäure bzw. Hyaluronat auch in Bakterienstämmen in pharmazeutischer Qualität hergestellt werden. Als Salze der Hyaluronsäure können bspw. Kalium-; Natrium-, und/oder Magnesiumhyaluronat verwendet werden. Besonders bevorzugt ist das Natrium-Hyaluronat.

Aufgrund dieser physikalischen Eigenschaften eignen sich wässrige Natrium-Hyaluronat-Lösungen und/oder Hyaluronsäure hervorragend als Gleit- und Schmiermittel mit guter Haftwirkung und verlängerter Verweilzeit auf den konjunktivalen und komealen Epithelien ohne Beeinträchtigung der Sehleistung.

Gemäß einer weiteren Ausführungsform weist die Hyaluronsäure und/oder das Hyaluronat ein Molekulargewicht auf, das in einem Bereich von 50.000 bis 10.000.000 Dalton, bevorzugt von etwa 250.000 bis 5.000.000 liegt. Besonders bevorzugt beträgt das Molekulargewicht der Hyaluronsäure bzw. des Hyaluronats 50.000 bis 4.000.000 Dalton. Äußerst bevorzugt weist die Hyaluronsäure bzw. das Hyaluronat ein Molekulargewicht von etwa 1.500.000 bis 3.500.000 Dalton auf. Die Hyaluronsäure und/oder das Hyaluronat werden vorzugsweise in einer Konzentration von 0,01 bis 1,0 Gew.-%, weiter bevorzugt von 0,05 bis 0,8 Gew.-%, besonders bevorzugt von 0,08 bis 0,4 Gew,-%, jeweils bezogen auf das Gesamtgewicht der phosphatfreien pharmazeutischen Zusammensetzung, verwendet.

Das hohe Molekulargewicht der Hyaluronsäure bzw. des verwendeten Hyaluronates wie bspw. Natrium-Hyaluronat bewirkt eine hohe Viskoelastizität bei niedriger Konzentration. In der Lösung liegen die Molekülketten in zufälliger Anordnung knäuelartig vor. Unter dem Einfluß der durch die Bewegung des Augenlides ausgeübten Scherkräfte richten sich die Makromoleküle in etwa parallel aus. Diese Änderung in der dreidimensionalen Struktur unter dem Einfluß von Scherkräften dürfte maßgeblich für die hervorragenden viskoelastischen Eigenschaften sein.

Unter Calcium-Chelatbildnem im Sinne der Erfindung werden vorzugsweise Stoffe verstanden, die eine Ausfällung von Calciumsalzen durch Komplexierung und/oder Bindung der Ca-Ionen verhindern.

Vorzugsweise ist der Calcium-Cheiatbildner der erfindungsgemäß zu verwendenden phosphatfreien pharmazeutischen Zusammensetzung aus der Gruppe ausgewählt, die aus Citratsalzen, Citronensäure, EDTA (Ethyiendiamintetraessigsäure), EGTA (Ethylenglykol-bis-(2-aminoethyl)-N,N,N',N'-tetraessigsäure) und Mischungen davon besteht.

Besonders bevorzugt umfaßt die erfindungsgemäß zu verwendende phosphatfreie pharmazeutische Zusammensetzung einen Citratpuffer. Die Verwendung von Citratpuffer ist besonders vorteilhaft, da der Citratpuffer zum einen als Puffersystem und zum anderen als Calciumchelatbildner wirkt, wodurch die Zugabe von weiteren Puffersystemen nicht mehr zwingend erforderlich ist.

Bei einer Ausführungsform umfaßt die erfindungsgemäß zu verwendende phosphatfreie pharmazeutische Zusammensetzung 0,04 bis 0,06 mg/ml Zitronensäure und 7,5 bis 9,5 mg/ml Trinatriumcitrat-Dihydrat, wobei der pH-Wert der erfingdungsgemäß zu verwendenden pharmazeutischen Zusammensetzung vorzugsweise 6,6 bis 7,8 beträgt.

Zur Herstellung des Citratpuffers können sowohl Citronensäure, primäre, sekundäre und/oder tertiäre Citrate verwendet werden. Als Citrate werden vorzugsweise Alkalimetallcitrate, weiter bevorzugt Natriumcitrat verwendet. Vorzugsweise werden Citronensäure, Natriumcitrat, Dinatriumcitrat und/oder Trinatriumcitrat verwendet.

Bei einer Ausführungsform ist der Calcium-Chelatbildner in einer Konzentration von 0,05 bis 10 Gew.%, weiter vorzugsweise von 0,1 bis 5 Ges.-%, besonders bevorzugt von 0,5 bis 3 Gew.-%, jeweils bezogen auf das Gesamtgewicht der pharmazeutischen Zusammensetzung, in der erfindungsgemäß zu verwendenden phosphatfreien pharmazeutischen Zusammensetzung enthalten. Bei einer weiteren Ausführungsform ist der Calcium-Chelatbildner in einer Konzentration von 0,8 bis 1,0 Gew.-%, jeweils bezogen auf das Gesamtgewicht der pharmazeutischen Zusammensetzung, in der erfindungsgemäß zu verwendenden phosphatfreien pharmazeutischen Zusammensetzung enthalten.

Die phosphatfreie pharmazeutische Zusammensetzung selbst ist Calciumionenfrei. Caiciumionen-frei im Sinne der Erfindung bedeutet, daß die phosphatfreie pharmazeutische Zusammensetzung weniger als 0,3 mmol/l CalciumIonen, vorzugsweise weniger als 0,1 mmol/l Calciumionen und besonders bevorzugt keine Calciumionen enthält.

Die erfindungsgemäß zu verwendende pharmazeutische Zusammensetzung verhindert aufgrund der Abwesenheit von Phosphationen und aufgrund des Vorhandenseins wenigstens eines Calcium-Chelatbildners, die Bildung von Calcium-P hosphat-Komplexen und/oder Calcium-Phosphat-Verbindungen und/oder oder anderen schwerlöslichen Ca-Verbindungen im Auge, die zu Ab- oder Einlagerungen auf oder in der Hornhaut führen können und mithin zu einer erheblichen Einschränkung des Sehvermögens durch Lichtstreuung an den Calcium-Phosphat-Komplexen und/oder Calcium-Phosphat-Verbindungen und/oder anderen schwerlöslichen Ca-Verbindungen. Unterschwerlöslichen CalciumVerbindungen werden vorzugsweise Verbindungen verstanden, die in der Hornhaut Ab- oder Einlagerungen bilden.

Der Calcium-Chelatbildner komplexiert physiologisch vorhandene Calciumionen und wirkt mithin der Entstehung schwer löslicher Calciumverbindungen, insbesondere von Calciumphosphatverbindungen, die zu Ab- oder Einlagerungen in der Hornhaut und/oder Bindehaut des Auges führen können, entgegen.

Die erfindungsgemäß zu verwendende pharmazeutische Zusammensetzung verhindert oder verringert eine Kalzifikation in der Hornhaut und/oder in der Bindehaut. Darüber hinaus können durch die erfindungsgemäße phosphatfreie Zusammensetzung vorzugsweise auch bereits abgelagerte Calcium-Phosphat-Komplexe und/oder Calcium-Phosphat-Verbindungen und/oder andere schwerlösliche CalciumVerbindungen von und/oder aus der Hornhaut gelöst werden.

Die hervorragenden Gleit- und Schmiermitteleigenschaften von Hyaluronsäure bzw. Hyaluronat wirken mit dem Calcium-Chelatbildner, welcher die Ablagerung und/oder Einlagerung von Calcium-haltigen Verbindungen oder Komplexen in der Hornhaut verhindert, synergistisch in dem mit der erfindungsgemäß zu verwendenden phosphatfreien pharmazeutischen Zusammensetzung behandelten Auge zusammen und ermöglichen die Bereitstellung einer außergewöhnlich gut verträglichen Benetzungslösung.

Überraschenderweise wurde auch festgestellt, daß die Viskosität von Hyaluronsäure-haltigen Lösungen und/oder Hyaluronat-haltigen Lösungen, welche keine Phosphationen enthalten, höher ist, als von Lösungen mit gleicher Menge an Hyaluronsäure und/oder Hyaluronat, welche Phosphationen enthalten. Vorteilhafterweise können somit in der erfindungsgemäß zu verwendenden phosphatfreien Zusammensetzung geringere Konzentrationen an Hyaluronsäure bzw. Hyaluronat eingesetzt werden, um eine langandauernde Benetzung des Auges zu erreichen. Da Hyaluron-säure bzw. Hyaluronat in pharmazeutischer Qualität sehr teuer ist, können erhebliche Kosten gespart werden.

Die phosphatfreie pharmazeutische Zusammensetzung wird konservierungsmittel-frei bereitgestellt. Konservierungsmittel können den präcornealen Film schädigen und zu einer Reduzierung der Anzahl der Mikrovilli und Mikroplicae der oberflächlichen Homhautepithelzellen führen. Somit kann allein die Langzeittherapie mit ophthalmologischen pharmazeutischen Zusammensetzungen, die Konservierungsmittel enthalten, zu einer Epithelschädigung führen, Insbesondere besitzt das weit verbreitete Benzalkoniumchlorid eine große Schädigungspotenz, Im Hinblick auf die gewünschte Therapie des Auges ist daher eine Reizung durch den Zusatz eines Konservierungsmittels zu vermeiden. Ferner behindern Konservierungsmittel die Regeneration der okulären Epithelien und sind deshalb bei der Behandlung bereits geschädigter Augen, z.B. beim Sicca-Syndrom, oder nach Traumata, z.B nach operativen Eingriffen, zu vermeiden.

Bei einer Ausführungsform umfasst die erfindungsgemäß zu verwendende pharmazeutische Zusammensetzung kein Panthenol, Panthothensäure, Heparin, Moxaverin und/oder deren Salze.

Unter dem Begriff Panthenol bzw. Panthothensäure sind weiterhin auch deren Derivate zu verstehen. Das Panthenol kann beispielsweise als Dexpanthenol vorliegen. Unter Panthothensäure werden auch deren Salze die Panthothenate, beispielsweise Natriumpantothenat, verstanden.

Unter dem Begriff "Heparin" sind "Mucopolysaccharide mit Heparinaktivität" zu verstehen. Unter einem "Mucopolysaccharid mit Heparin-Aktivität" wird vorzugsweise jedes Mucopolysaccharid bzw. Glucosaminoglykan verstanden, das eine dem Heparin vergleichbare biologische bzw. physiologische Aktivität aufweist. Das Mucopolysaccharid mit Heparin-Aktivität ist beispielsweise aus der Gruppe ausgewählt, die aus Heparinoiden, humanem Heparin, tierischem Heparin, rekombinantem Heparin, chemisch modifiziertem Heparin, enzymatisch modifiziertem Heparin, trunkiertem Heparin, niedermolekularem Heparin, Heparansulfiat und Mischungen davon besteht.

Gemäß einer bevorzugten Ausführungsform sind die vorgenannten Arzneimittel Panthenol, insbesondere Dexpanthenol, Panthothensäure, Heparin, Moxaverin und/oder deren Salze nicht in der erfindungsgemäß zu verwendenden phosphatfreien pharmazeutischen Zusammensetzung enthalten.

Es hat sich völlig überraschend herausgestellt, daß eine phosphatfreie Zusammensetzung, die kein Panthenol, insbesondere kein Dexpanthenol, enthält, zur Behandlung von Epitheldefekten in der Hornhaut und/oder Bindehaut des Auges sehr eignet ist. Obgleich der Wirkstoff Panthenol, insbesondere Dexpanthenol, umfangreich bei der Heilung von Wunden verwendet wird, hat sich völlig unerklärlich herausgestellt, daß im Falle von Verletzungen der Hornhaut und/oder Bindehaut des Auges auch dann eine sehr gute Heilung eintritt, wenn die phosphatfreie, pharmazeutische Zusammensetzung kein Panthenol, insbesondere kein Dexpanthenol, enthält. Somit ermöglicht die vorliegende Erfindung die Bereitstellung einer nurwenige Komponenten umfassenden pharmazeutischen Zusammensetzung mit hervorragendem Wirkungsprofl. Die erfindungsgemäße Zusammensetzung kann mithin preisgünstiger hergestellt werden, da auf den Wirkstoff Dexpanthenol verzichtet werden kann, ohne jedoch eine schlechtere Heilungswirkung zu haben.

Bei Verwendung der phosphatfreien pharmazeutischen Zusammensetzung kommt es ferner nicht zu Ein- und/oder Ablagerung von schwer löslichen Calciumverbindungen wie Calciumphosphatverbindungen. Bei Verwendung herkömmlicher Phosphatpuffer-haltiger Augenpräparate kommt es hingegen zu Einlagerungen und/oder Ablagerungen, insbesondere von Calciumphosphatverbindungen, auch in die heilende Hornhaut und/oder Bindehaut des Auges.

Somit stellt die vorliegende Erfindung einen großen Fortschritt dar, da bei Verwendung der Zusammensetzung diese Ein- und/oder Ablagerungen in und/oder auf der Hornhaut und/oder Bindehaut des Auges zuverlässig vermieden werden und zugleich eine sehr gute Heilung von Hornhaut und/oder Bindehaut des Auges erhalten wird.

Gemäß einer bevorzugten Ausführungsform besteht die phosphatfreie pharmazeutische Zusammensetzung aus mindestens einem Calcium-Chelatbildner und mindestens einem ophthalmologisch verträglichen Viskositätsregler sowie optional einem pharmazeutischen Hilfsstoff oder mehreren pharmazeutischen Hilfsstoffen. In diesem Fall bedeutet "aus mindestens einem", daß die phosphatfreie pharmazeutische Zusammensetzung aus einem odermehreren Calcium-Chelatbildnern und aus einem oder mehreren ophthalmologisch verträglichen Viskositätsreglern und optional aus einem oder mehreren pharmazeutischen Hilfsstoffen besteht.

Gemäß einer besonders bevorzugten Ausführungsform besteht die phosphatfreie pharmazeutische Zusammensetzung aus Citronensäure und/oder Citratsalzen und Hyaluronat und/oder Hyaluronsäure sowie optional einem pharmazeutischen Hilfsstoff oder mehreren pharmazeutischen Hilfsstoffen.

Bei einer Ausführungsform umfasst die erfindungsgemäß zu verwendende pharmazeutische Zusammensetzung keinen weiteren Arzneistoff neben dem Calcium-Chelatbildner, Hyaluronsäure und/oder Hyaluronaten. Unter Arzneistoffe im Sinne der Erfindung werden insbesondere Stoffe verstanden, die im lebenden Organismus die Verhütung, Heilung und/oder Linderung von Krankheiten bewirken.

Bevorzugt wird zur Lagerung und Abgabe einer erfindungsgemäßen Konservierungsmittel-freien pharmazeutischen Zusammensetzung das in "PTA heute" 1996, Nr. 12, Seite 1230 bis 1232 beschriebene COMOD^{®}-System, verwendet, das eine sterile Lagerung und mehrfache Abgabe der erfindungsgemäß zu verwendenden, phosphatfreien pharmazeutischen Zusammensetzung erlaubt. Selbstverständlich können auch herkömmliche Einzeldosisbehältnisse verwendet werden, die nach Gebrauch weggeworfen werden.

Bei einer Ausführungsform kann die phosphatfreie pharmazeutische Zusammensetzung weitere pharmazeutische Hilfsstoffe umfassen, weiche ophthalmologisch verträglich sind Die pharmazeutischen Hilfsstoffe sind dabei vorzugsweise phosphationenfrei.

Vorzugsweise sind die pharmazeutischen Hilfsstoffe aus der Gruppe ausgewählt, die aus anorganischen Puffersubstanzen, organischen Puffersubstanzen, anorganischen Salzen, organischen Salzen, Viskositätsreglern, Lösungsmitteln, Lösungsvermittler, Lösungsbeschleuniger, Salzbildnern, Salzen, Viskositäts- und Konsistenzbeeinflussern, Gelbildnern, Emulgatoren, Solubilisatoren, Benetzem, Spreizmitteln, Anti-Oxidantien, Konservierungsmitteln, Füll-und Trägerstoffen, Osmolaritätsreglem sowie Mischungen davon besteht.

Weiter ist es bevorzugt, daß die phosphatfreie pharmazeutische Zusammensetzung in Form einer Lösung, Suspension, Emulsion, eines Gels, einer Salbe, Paste, eines Pulvers, Granulats oder einer Tablette vorliegt. Die phosphatfreie pharmazeutische Zusammensetzung ist bevorzugt ein Ophthalmikum, weiter bevorzugt ein Ophthalmikum zur topischen Anwendung.

Bei der Bereitstellung der phosphatfreien pharmazeutischen Zusammensetzung in Form von Augensalben bzw, Augengelen wird diese bspw. in Vaseline oder Paraffin mit und ohne Emulgatorzusatz wie bspw. Cholesterin, Wollwachs, Wollwachsalkohole, Cetanol etc. bereitgestellt.

Gemäß einer bevorzugten Ausführungsform liegt die phosphatfreie pharmazeutische Zusammensetzung in der Form einer Lösung vor, so daß diese bspw. in der Form von Augentropfen oder eines Augensprays auf die Oberfläche des Auges aufgebracht werden kann.

Bei einer Ausführungsform liegt die Osmolarität der erfindungsgemäßen phosphatfreien pharmazeutischen Zusammensetzung bei 100 bis 900 mOsm/l.

Die vorzugsweise wäßrigen Lösungen sind dabei gemäß einer bevorzugten Ausführungsform, bezogen auf die Tränenflüssigkeit, isotone Lösungen. Bei isotonen Lösungen liegt die Osmolarität vorzugsweise bei 200 bis 350 mOsm/l, vorzugsweise bei 300 mOsm/l. Gemäß einer weiteren bevorzugten Ausführungsform ist die erfindungsgemäß zu verwendende phosphatfreie pharmazeutische Zusammensetzung hypoosmolar. In diesem Fall kann die Osmolarität beispielsweise etwa 160-180 mOsm/l betragen. Eine hypoosmolare Lösung findet insbesondere dann Anwendung, wenn eine abnorm hohe Osmolarität eines Tränenfilms bei einem Patienten mit trockenen Augen ausgeglichen werden muß, In Abhängigkeit von dem zu behandelnden Krankheitsbild kann auch eine hypertone Lösung vorteilhaft sein. Die pharmazeutische Zusammensetzung kann dabei auch eine besonders hohe Osmolarität von 700 bis 900 mOsm/l aufweisen.

Zur Isotonisierung der wäßrigen Lösung werden bevorzugt Natriumchlorid, Borsäure, Sorbitol, Glycerin, etc. verwendet.

Der pH-Wert der wäßrigen Lösung liegt bevorzugt in einem Bereich von pH 5 bis 9, weiter bevorzugt in einem Bereich von pH 6,8 bis 7,6, besonders bevorzugt in einem Bereich von pH 7,2 bis 7,4. Zur Einstellung des pH-Werts können Pufferlösungen wie beispielsweise Acetat-Puffer, Acetat-Borat-Puffer und Borat-Puffer verwendet werden. Erfindungsgemäß werden jedoch keine Phosphatpuffer verwendet.

Selbstverständlich ist es möglich, daß die erfindungsgemäß zu verwendende phosphatfreie pharmazeutische Zusammensetzung in der Form eines Feststoffes vorliegt, der vor einerApplikation zunächst in einer wässrigen Lösung wie bspw. einer Pufferlösung aufgelöst wird. Nach Auflösung eines Feststoffes, bspw. in einer wässrigen Pufferlösung kann diese Lösung steril filtriert werden und kann dann als Augenspray bzw. Augentropfen auf die Augenoberfläche aufgebracht werden. Bevorzugt liegen Feststoff und Lösungsmittel bei der getrennten Aufbewahrung bereits in steriler Form vor, so daß ein Sterilfiltrieren nach Herstellung der Lösung nicht erfordedich ist. Der Anwender kann somit nach Herstellung der Mischung bzw. Lösung die phosphatfreie pharmazeutische Zusammensetzung direkt applizieren.

Beim Bereitstellen einer phosphatfreien pharmazeutischen Zusammensetzung in Form eines Feststoffs wie bspw. eines Pulvers, Puders, Granulats oder einer Tablette umfaßt die erfindungsgemäße phosphatfreie pharmazeutische Zusammensetzung bevorzugt Natrium-Citrat und/oder Citronensäure sowie Hyaluronsäure und/oder Natrium-Hyaluronat, da diese Verbindungen in Wasser sehr gut löslich sind.

Grundsätzlich kann die erfindungsgemäß zu verwendende phosphatfreie pharmazeutische Zusammensetzung auch in Form von Augentabletten in den Bindehautsack eingebracht werden. Unter Einwirkung von Tränenflüssigkeit löst sich die Augentablette rasch auf.

Bevorzugt erfolgt jedoch die Applikation der phosphatfreien pharmazeutischen Zusammensetzung in der Form von Augentropfen, Augensprays und/oder Augengelen.

Vor der Applikation der Lösung oder des Feststoffs werden die Verbindungen in den gewünschten Mengenverhältnissen miteinander vermengt und unter Zugabe von Wasser bzw, wässrigen Pufferlösungen aufgelöst und nachfolgend steril filtriert.

Erfindungsgemäß wird die phosphatfreie pharmazeutische Zusammensetzung bei einer Ausführungsform zur Behandlung und/oder Prävention der Kalzifikation bzw. der bandförmigen Homhauttrübung verwendet. Die Kalzifikation tritt insbesondere an Epitheldefekten auf, die unterschiedliche Ursachen haben können.

Beispielsweise sind die Epitheldefekte aus derGruppe, bestehend aus durch mechanische und/oderchemische Einwirkungen hervorgerufene Epitheldefekte, Epitheldefekte nach chirurgischen Eingriffen, Epitheldefekte durch Benetzungsstörungen der Augenoberfläche, Epitheldefekte durch Langzeitbehandlung mit Konservierungsmittel-haltigen pharmazeutischen Zusammensetzungen oder mit Kontaktlinsen, ausgewählt. Epitheldefekte, beispielsweise Schnitte, Verätzungen und Läsionen, ermöglichen die Ein- und/oder Anlagerung von schwer löslichen Calciumverbindungen, insbesondere von Calciumphosphat, in die und/oder auf der Hornhaut des Auges,

Beispielsweise sind die Benetzungsstörungen aus der Gruppe, bestehend aus Sjögren-Syndrom, Sicca-Syndrom und Benetzungsstörungen bei Kontaktlinsenträgem, ausgewählt. Benetzungsstörungen am Auge führen zu einer erhöhten Reibung am Auge oder auf der Oberfläche des Auges. Bereits durch diese Reibung kann es zu Epitheldefekten kommen, welche das Risiko einer Kalzifikation signifikant erhöhen.

Die durch mechanischen Einwirkungen bedingten Epitheldefekte können beispielsweise unfallbedingte Verletzungen sein.

Die durch chemische Einwirkungen hervorgerufene Epitheldefekte können beispielsweise durch Verätzungen mit Säuren und/oder Laugen entstehen.

Bei einer Ausführungsform sind die chirurgischen Eingriffe aus der Gruppe, bestehend aus chirurgischen Eingriffen am vorderen Augenabschnitt, Kataraktextraktion mit Linsenimplantation, refraktiv-chirurgischen Eingriffen, Eingriffen an der Hornhaut und Hornhauttransplantationen, ausgewählt.

Bei einer Ausführungsform wird die erfindungsgemäß zu verwendende ophthalmologische phosphatfreie pharmazeutische Zusammensetzung bei der Langzeitbehandlung, insbesondere von chronischen Erkrankungen am Auge, eingesetzt. Insbesondere wird die erfindungsgemäß zu verwendende ophthalmologische phosphatfreie pharmazeutische Zusammensetzung bei der Langzeitbehandlung von Allergien und/oder Glaukomen eingesetzt. Unter Langzeitbehandlung im Sinne der Erfindung wird eine Behandlung von vorzugsweise mehr als einem Jahr, besonders bevorzugt von mehr als einem Monat, äußerst bevorzugt von mehr als einer Woche verstanden.

Vorzugsweise ist bei der erfindungsgemäßen Verwendung die phosphatfreie Zusammensetzung calciumfrei und/oder dexpanthenolfrei.

Gemäß einer weiteren bevorzugten Ausführungsform wird bei der erfindungsgemäßen Verwendung der Viskositätsregler aus der Gruppe ausgewählt, die aus Chondroitinsulfat, Polyacrylamid, Polyacrylsäure, Polyacrylharze, Polyethylenglykol, Cellulosederivate, Polysacchariden, Polyvinylalkohol, Polyvinylpyrrolidon, Hyaluronsäure, Hyaluronaten, Derivate davon und Mischungen davon besteht.
Ferner kann die erfindungsgemäße phosphatfreie pharmazeutische Zusammensetzung als künstliche Tränen, beispielsweise zur Nachbenetzung bei Kontaktlinsenträgem, verwendet werden.

Im übrigen wird in bezug auf die erfindungsgemäße Verwendung auf die obigen Ausführungen zur Zusammensetzung verwiesen; die entsprechend auch bei der erfindungsgemäßen Verwendung gelten.

### Beispiele

### Beispiel 1

**1 ml Augentropfen enthält:**

| | |
|---|---|
| Natriumhyaluronat (MG: 1,5 x 10⁶ - 3,5x10⁶ Da) | 1,0 mg |
| Sorbit | 32,0 mg |
| Citronensäure, wasserfrei | 0,05 mg |
| Natriumcitrat x 2 H₂O | 8,5 mg |
| Wasser für Injektionszwecke ad pH 6,8 bis 7,6 | 1,0 ml |

### Beispiel 2

| | |
|---|---|
| Natriumhyaluronat (MG: 1,5 x 10⁶ - 3,5x10⁶ Da) | 5,0 mg |
| Citronensäure wasserfrei | 29,5 mg |
| Natriumcitrat x 2 H₂O | 500 mg |
| Wasser für Injektionszwecke | ad 50 ml |
| pH 6,25 | |
| 109 mOsm/kg | |

### Beispiel 3

| | |
|---|---|
| Natriumhyaluronat (MG:1,5 x 10⁶ - 3,5x10⁶ Da) | 10,0 mg |
| Citronensäure wasserfrei | 0,3 mg |
| Natriumcitrat x 2 H₂O | 50 mg |
| Glycerol wasserfrei | 2500 mg |
| Wasser für Injektionszwecke | ad 100 ml |
| pH 7,11 | |
| 270 mOsml/kg | |

Bei dem gemäß Beispiel 1 bis 3 eingesetzten Natriumcitrat handelt es sich um Trinatriumcitrat Dihydrat in Arzneibuchqualität, Die Rezepturen gemäß Beispiel 1 bis 3 können weitere Bestandteile zur Isotonisierung und zur Isohydrierung enthalten.

### Beispiel 4

In diesem Beispiel wurde die Heilungswirkung von Augentropfen unter Berücksichtigung von Verkalkungs- bzw. Kalzifikationserscheinungen in der Hornhaut von phosphathaltigen und erfindungsgemäßen phosphatfreien Augentropfen miteinander verglichen.

Die zum Vergleich verwendeten phosphathaltigen Augentropfen hatten dabei folgende Zusammensetzung:

| | |
|---|---|
| Natriumhyaluronat (MG: 1,5 x 10⁶ - 3,5 x 10⁶ Da) | 1 mg |
| Sorbit | 27 mg |
| Phosphatpuffer pH 7,2 | 50 mM |
| Wasser für Injektionszwecke | ad 1 ml |

Die erfindungsgemäßen phosphatfreien Augentropfen hatten dabei folgende Zusammensetzung:

| | |
|---|---|
| Natriumhyaluronat (MG:1,5 x 10⁶-3,5 x 10⁶ Da) | 1 mg |
| Sorbit | 32 mg |
| Citronensäure wasserfrei | 0,05 mg |
| Natriumcitrat x 2 H₂O | 8,5 mg |
| Wasser für Injektionszwecke | ad 1 ml |
| pH,7,2 | |

Die Heilungswirkung unter Berücksichtigung von Verkalkungs- bzw. Kalzifikationserscheinungen in der Hornhaut des Auges wurde an Hornhäuten untersucht, die frisch mit Kohlendioxidgas getöteten Kaninchen nach Ausschälung der Augen entnommen waren. Nach Entfernung der Retina, Choroidea, Linse sowie Iris wurden die Hornhäute mit Resten der Sklera in einer Kulturkammer in einer Haltvorrichtung fixiert. Die hinter der Comea (Hornhaut) liegende vordere Augenkammer wurde in der Kulturkammer sodann mit Kulturmedium vorsichtig befüllt.

Als Kulturmedium, wurde serumfreies Earle's MEM T031-05 der Fa. Biochrom, Berlin, Deutschland verwendet. Der pH-Wert des Kulturmediums wurde unter Zugabe von NaHCO₃ auf pH 7,2 eingestellt.

Das Kulturmedium wurde im Durchfluß mit einer Flußrate von 10 µl/Minute kontinuierlich ausgetauscht. Das durchschnittliche Volumen der artifiziellen Vorderkammer betrug in Abhängigkeit von der Krümmung der Hornhaut 0,5 bis 0,9 ml. Ein Kulturmediumaustausch erfolgte mithin über einen Zeitraum von 50 bis 90 Minuten.

Die Kultivierung der Hornhäute erfolgte unter sterilen Bedingungen bei 32°C unter 100 % Luftfeuchtigkeit in einem Brutschrank.

Um die Heilungswirkung unter Berücksichtigung von Verkalkungserscheinungen an den Hornhäuten zu untersuchen, wurde unter Verwendung eines Zahnarztschleifers (Arkansasschleifers 638XF, Fa. Meisinger, Deutschland) die Hornhautoberfläche in Form von vier kleinen Erosionen mit je ca. 0,2 bis 1,5 mm2 Fläche verletzt.

Über dem Apex Cornea wurde eine Tropfkanüle angeordnet, so daß die Hornhaut zentrisch betropft wurde. Unter Verwendung einer Mikropumpe wurde je Stunde ein Tropfen Flüssigkeit mit einem Volumen von jeweils circa 30 µl des Kulturmediums, der phosphathaltigen Augentropfen oder der phosphatfreien Augentropfen aufgebracht.

Das Auftropfen von Kulturmedium diente als Kontrolle zum Vergleich der mit den phosphathaltigen bzw. phosphatfreien Augentropfen behandelten Hornhäuten.

Um die Verkalkungserscheinungen in der Hornhaut untersuchen zu können, wurden die Hornhäute ferner mit einer Calciumchlorid-haltigen Kochsalzlösung mit einer CaCl₂-Konzentration von 14,58 mmol/l mit einem Tropfen (30 µl) /Std. betropft. Das Auftropfen der Calciumchlorid-haltigen physiologischen Kochsalzlösung diente der Simulation einerTränensekretion. Die Konzentration an freien Calciumionen entspricht dabei dem Calciumgehalt in dersekretierten Tränenflüssigkeit.

Das Auftropfen von Augentropfen bzw. Calciumchloridlösung erfolgte alternierend jeweils in 30-minütigem Abstand.

Die untersuchten Hornhäute waren in den Versuchen vollständig lebensfähig. Die Lebensfähigkeit wurden anhand des pH-Wertes sowie der Glucose- und Lactatkonzentration im Medium der Kulturkammern gemessen. Sämtliche Werte waren über die Meßdauer konstant.

Die Meßdauer betrug jeweils 4 Tage.

Das Ergebnis bezüglich der Verkalkung und Heilung der Hornhäute ist in Tabelle 1 zusammengefaßt.

**Tabelle 1**

| **Ergebnis Verkalkung und Heilung** | | | | | |
|---|---|---|---|---|---|
| **Lauf Nr** | **Hornhaut Nr.** | **Typ der Exposition** | **Stärke der Verkalkung** | **Verkalkung aufgetreten (≧1)** | **Heilung [%]** |
| 1 | HH1 | Medium | 1 | ja | 49,7 |
| 1 | HH2 | phosphatfrei | 0 | nein | 89,8 |
| 1 | HH3 | phosphathaltig | 4 | ja | 22 |
| 2 | HH7 | Medium | 4 | ja | 97,8 |
| 2 | HH8 | phosphatfrei | 0 | nein | 100 |
| 2 | HH9 | phosphathaltig | 3 | Ja | 92 |
| 3 | HH13 | Medium | 3 | Ja | n.d. |
| 3 | HH16 | phosphathaltig | 4 | Ja | n.d. |
| 3 | HH17 | phosphatfrei | 0 | nein | 94,8 |
| Erläuterung der Tabelle: | | | | | |
| 1.) n.d.: nicht bestimmt. | | | | | |
| 2.) Die Angaben der Heilung in % gibt die Größe der ausgeheilten Fläche bezogen auf die ursprüngliche Fläche der Hornhautverletzung an. | | | | | |
| 3.) Die Stärke der Verkalkung wurde anhand der Wertzahlen 1 bis 5 angegeben, wobei die Bewertung mit dem bloßen Auge erfolgte und die Wertzahlen für folgende Bewertungen stehen: | | | | | |
| 0: keine Verkalkungserscheinungen | | | | | |
| 1: erste Verkalkungen sind mit dem bloßen Auge erkennbar | | | | | |
| 2: leichte Verkalkung | | | | | |
| 3: mittelstarke Verkalkung | | | | | |
| 4: starke Verkalkung | | | | | |
| 5: vollständige Verkalkung | | | | | |

### Figuren:

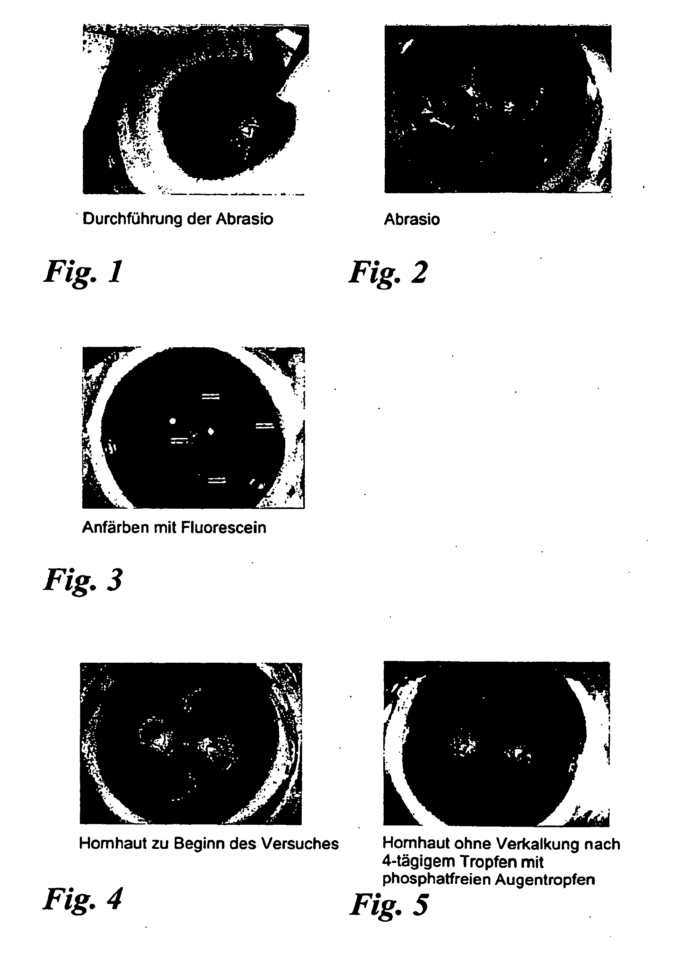

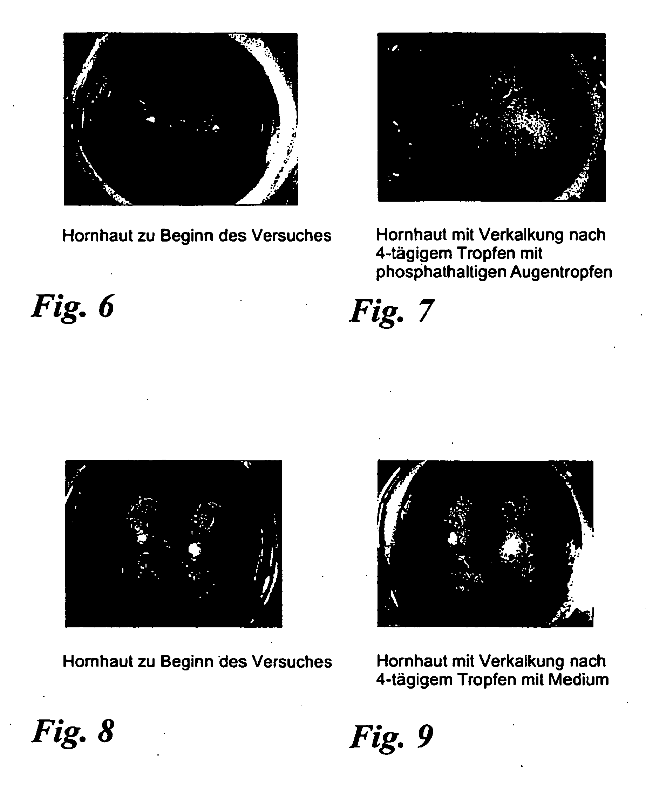

In den Figuren 1 bis 9 sind photomikroskopische Aufnahmen von behandelten und unbehandelten Hornhäuten bei Durchlichtbetrachtung gezeigt. Zu sehen sind jeweils die unter Kultivierungsbedingungen gehaltenen und eingespannten sowie behandelten Hornhäute von Kaninchenaugen.
In Fig. 1 ist gezeigt, wie die Hornhautverletzung unter Verwendung eines Zahnarztschleifers aufgebracht wird.
In Fig. 2 sind die Verletzungen der Hornhautoberfläche, auch als Abrasio bezeichnet, gezeigt.
In Fig. 3 sind die der Hornhaut des Auges zugefügten Verletzungen unter Anfärbung mit Fluorescein besser sichtbar gemacht.
In Fig. 4 ist die verletzte Hornhaut zu Beginn des Versuches mit den phosphatfreien Augentropfen aus Beispiel 4 gezeigt.
In Fig. 5 ist die Hornhaut aus Fig. 4 nach 4-tägigem Betropfen mit phosphatfreien Augentropfen gezeigt. Aus Fig. 5 ist es ersichtlich, daß es zu keinerlei Verkalkung der Hornhautoberfläche nach 4-tägigem Betropfen mit den phosphatfreien Augentropfen kam.
In Fig. 6 ist die Hornhaut zu Beginn des Versuches mit den phosphathaltigen Augentropfen aus Beispiel 4 gezeigt.
In Fig. 7 ist die Hornhaut aus Fig. 6 nach 4-tägigem Betropfen mit den phosphathaltigen Augentropfen gezeigt. Aus Fig. 7 ist ersichtlich, daß es nach 4-tägigem Betropfen mit den phosphathaltigen Augentropfen zu einer starken Verkalkung der Hornhautoberfläche kam.
In Fig. 8 ist die Hornhaut zu Beginn des Kontrollversuches mit Kulturmedium, wie in Beispiel 4 beschrieben, gezeigt.
In Fig. 9 ist die Hornhautoberfläche aus Fig. 8 nach 4-tägigem Betropfen mit Kulturmedium zu sehen. Wie aus Fig. 9 ersichtlich ist, kam es zu einer mittelstarken bis starken Verkalkung der Hornhautoberfläche nach 4-tägigem Betropfen mit Kulturmedium.

### Ergebnis:

Es hat sich völlig überraschend gezeigt, daß es bei Behandlung von Epitheldefekten in der Hornhaut und/oder Bindehaut des Auges mit phosphatfreien Augentropfen, die einen Calcium-Chelatbildner enthalten, zu keiner Verkalkung der Hornhautoberfläche, d.h. insbesondere zu keiner Einlagerung von Calciumphosphat-Verbindungen, kommt.

Ferner hat sich überraschend herausgestellt, daß die erfindungsgemäße phosphatfreie pharmazeutische Zusammensetzung, auch bei Abwesenheit von Dexpanthenol, das üblicherweise in breitem Umfang zur Wundheilung verwendet wird, eine sehr gute Heilung einer verletzten Hornhaut des Auges bewirkt.

Aus Tabelle 1 ist ersichtlich, daß die über die zwei bzw. drei Versuchsläufe gemittelten Heilungsraten im Falle von Kulturmedium 73,8 %, von den phosphathaltigen Augentropfen 57 % und von den phosphatfreien Augentropfen 94,9 % betrugen.

Im Unterschied zur Behandlung mit den phosphathaltigen Augentropfen, bei deres zu einerstarken Verkalkung der Horn hautoberfläche mit einer durchschnittlichen Bewertungszahl von etwa 4 kam, konnte keine Verkalkung der Hornhautoberfläche im Falle der Verwendung von phosphatfreien Augentropfen festgestellt werden.

## Patentansprüche

1. Verwendung von mindestens einem Calcium-Chelatbildner und mindestens einem ophthalmologisch verträglichen Viskositätsregler zur Herstellung einer phosphatfreien und konservierungsmittelfreien pharmazeutischen Zusammensetzung zur Behandlung und/oder Prävention von Epitheldefekten in der Hornhaut und/oder Bindehaut des Auges.

2. Verwendung nach Anspruch 1,
**dadurch gekennzeichnet,**
**dass** die Epitheldefekte aus der Gruppe, bestehend aus durch mechanische und/oder chemische Einwirkungen hervorgerufene Epitheldefekte, Epitheldefekte nach chirurgischen Eingriffen, Epitheldefekte durch Benetzungsstörungen der Augenoberfläche, Epitheldefekte durch Langzeitbehandlung mit Konservierungsmittel-haltigen pharmazeutischen Zusammensetzungen oder mit Kontaktlinsen, ausgewählt sind.

3. Verwendung nach einem Anspruch 1 oder 2,
**dadurch gekennzeichnet,**
**dass** die phosphatfreie pharmazeutische Zusammensetzung zur Langzeitanwendung eingesetzt wird.

4. Verwendung nach Anspruch 3,
**dadurch gekennzeichnet,**
**dass** die Langzeitanwendung bei Glaukomen und bei Allergien erfolgt.

5. Verwendung nach einem der Ansprüche 1 bis 4,
**dadurch gekennzeichnet,**
**dass** Ein- und/oder Ablagerungen von schwerlöslichen Calciumverbindungen in der Hornhaut und/oder Bindehaut des Auges, insbesondere eine Kalzifikation, verhindert und/oder reduziert werden.

6. Verwendung nach einem der Ansprüche 2 bis 5,
**dadurch gekennzeichnet,**
**dass** die Benetzungsstörungen aus der Gruppe, bestehend aus Sjögren-Syndrom, Sicca-Syndrom und Benetzungsstörungen des Auges bei Kontaktlinsenträgern, ausgewählt sind.

7. Verwendung nach einem der Ansprüche 2 bis 6,
**dadurch gekennzeichnet,**
**dass** die chirurgischen Eingriffe aus der Gruppe, bestehend aus chirurgischen Eingriffen am vorderen Augenabschnitt, Kataraktextraktion mit Linsenimplantation, refraktiv-chirurgischen Eingriffen, Eingriffen an der Hornhaut und Hornhauttransplantationen, und Eingriffen an der Bindehaut ausgewählt sind.

8. Verwendung nach einem der Ansprüche 1 bis 7,
**dadurch gekennzeichnet,**
**dass** die phosphatfreie pharmazeutische Zusammensetzung Calciumionen frei ist.

9. Verwendung nach einem der Ansprüche 1 bis 8,
**dadurch gekennzeichnet,**
**dass** die Menge an Viskositätsregler etwa 0,005 Gew.-% bis etwa 5 Gew.-%, bevorzugt etwa 0,01 Gew.-% bis etwa 1 Gew.-%, jeweils bezogen auf das Gesamtgewicht der phosphatfreien pharmazeutischen Zusammensetzung, beträgt.

10. Verwendung nach einem der Ansprüche 1 bis 9,
**dadurch gekennzeichnet,**
**dass** der Viskositätsregler aus der Gruppe ausgewählt wird, die aus Chondroitinsulfat, Polyacrylamid, Polyacrylsäure, Polyacrylharze, Polyethylenglykol, Cellulosederivate, Polysacchariden, Polyvinylalkohol, Polyvinylpyrrolidon, Hyaluronsäure, Hyaluronaten, Derivate davon und Mischungen davon besteht.

11. Verwendung nach Anspruch 10,
**dadurch gekennzeichnet,**
**dass** die Hyaluronsäure, das Hyaluronat und/oder deren Derivate ein Molekulargewicht aufweisen, das in einem Bereich von etwa 50.000 bis etwa 10.000.000 Dalton, bevorzugt von etwa 250.000 bis etwa 5.000.000 Dalton, liegt.

12. Verwendung nach Anspruch 10 oder 11,
**dadurch gekennzeichnet,**
**dass** das Hyaluronat Natrium-Hyaluronat ist.

13. Verwendung nach einem der Ansprüche 1 bis 12,
**dadurch gekennzeichnet,**
**dass** der Calcium-Chelatbildner aus der Gruppe ausgewählt wird, die aus Citratsalzen, Citronensäure, EDTA, EGTA und Mischungen davon besteht.

14. Verwendung nach einem der Ansprüche 1 bis 13,
**dadurch gekennzeichnet,**
**dass** die pharmazeutische Zusammensetzung weitere pharmazeutische Hilfsstoffe enthält.

15. Verwendung nach Anspruch 14,
**dadurch gekennzeichnet,**
**dass** die pharmazeutischen Hilfsstoffe aus der Gruppe ausgewählt werden, die aus anorganischen Puffersubstanzen, organischen Puffersubstanzen, anorganischen Salzen, organischen Salzen, Viskositätsreglern, Lösungsmitteln, Lösungsvermittlern, Lösungsbeschleunigern, Salzbildnern, Viskositäts- und Konsistenzbeeinflussern, Gelbildnern, Emulgatoren, Solubilisatoren, Benetzern, Spreizmitteln, Anti-Oxidantien, Konservierungsmitteln, Füll- und Trägerstoffen, Osmolaritätsreglern sowie Mischungen davon besteht.

16. Verwendung nach einem der Ansprüche 1 bis 15,
**dadurch gekennzeichnet,**
**dass** die phosphatfreie pharmazeutische Zusammensetzung in Form einer Lösung, von Tropfen, eines Sprays, einer Suspension, Emulsion, eines Gels, einer Salbe, Paste, eines Puders, Pulvers, Granulats oder einer Tablette vorliegt.

17. Verwendung nach einem der Ansprüche 1 bis 16,
**dadurch gekennzeichnet,**
**dass** die phosphatfreie pharmazeutische Zusammensetzung kein Moxaverin, Heparin, Panthenol und/oder Pantothensäure enthält.

18. Verwendung nach einem der Ansprüche 1 bis 17,
**dadurch gekennzeichnet,**
**dass** die phosphatfreie pharmazeutische Zusammensetzung keinen weiteren Arzneistoff enthält.

19. Verwendung nach einem der Ansprüche 11 bis 18,
**dadurch gekennzeichnet,**
**dass** die phosphatfreie pharmazeutische Zusammensetzung aus mindestens einem Calcium-Chelatbildner und mindestens einem ophthalmologisch verträglichen Viskositätsregler sowie optional einem pharmazeutischen Hilfsstoff oder mehreren pharmazeutischen Hilfsstoffen besteht.

## Claims

1. Use of at least one calcium chelating agent and at least one ophthalmologically compatible viscosity regulator for the production of a phosphate-free and preservative-free pharmaceutical composition for the treatment and/or prevention of epithelial defects in the cornea and/or conjunctiva of the eye.

2. Use according to claim 1, **characterized in that** the epithelial defects are selected from the group consisting of epithelial defects produced by mechanical and/or chemical actions, epithelial defects after surgical interventions, epithelial defects due to wetting disorders of the surface of the eye, epithelial defects due to long-term treatment with preservative-containing pharmaceutical compositions or with contact lenses.

3. Use according to claim 1 or 2, **characterized in that** the phosphate-free pharmaceutical compositions is employed for long-term application.

4. Use according to claim 3, **characterized in that** the long-term use takes place in glaucoma and in allergies.

5. Use according to one of claims 1 to 4, **characterized in that** incorporations and/or deposits of poorly soluble calcium compounds in the cornea and/or conjunctiva of the eye, in particular calcification, are prevented and/or reduced.

6. Use according to one of claims 2 to 5, **characterized in that** the wetting disorders are selected from the group consisting of Sjögren syndrome, sicca syndrome and wetting disorders of the eye in contact lens wearers.

7. Use according to one of claims 2 to 6, **characterized in that** the surgical interventions are selected from the group consisting of surgical interventions on the anterior section of the eye, cataract extraction with lens implantation, refractive-surgical interventions, interventions on the cornea and corneal transplants, and interventions on the conjunctiva.

8. Use according to one of claims 1 to 7, **characterized in that** the phosphate-free pharmaceutical composition is free from calcium ions.

9. Use according to one of claims 1 to 8, **characterized in that** the amount of viscosity regulator is approximately 0.005% by weight to approximately 5% by weight, preferably approximately 0.01% by weight to approximately 1% by weight, in each case based on the total weight of the phosphate-free pharmaceutical composition.

10. Use according to one of claims 1 to 9, **characterized in that** the viscosity regulator is selected from the group which consists of chondroitin sulfate, polyacrylamide, polyacrylic acid, polyacrylic resins, polyethylene glycol, cellulose derivatives, polysaccharides, polyvinyl alcohol, polyvinylpyrrolidone, hyaluronic acid, hyaluronates, derivatives thereof and mixtures thereof.

11. Use according to claim 10, **characterized in that** the hyaluronic acid, the hyaluronate and/or their derivatives have a molecular weight which is in a range from approximately 50 000 to approximately 10 000 000 daltons, preferably from approximately 250 000 to approximately 5 000 000 daltons.

12. Use according to claim 10 or 11, **characterized in that** the hyaluronate is sodium hyaluronate.

13. Use according to one of claims 1 to 12, **characterized in that** the calcium chelating agent is selected from the group which consists of citrate salts, citric acid, EDTA, EGTA and mixtures thereof.

14. Use according to one of claims 1 to 13, **characterized in that** the pharmaceutical composition contains further pharmaceutical excipients.

15. Use according to claim 14, **characterized in that** the pharmaceutical excipients are selected from the group which consists of inorganic buffer substances, organic buffer substances, inorganic salts, organic salts, viscosity regulators, solvents, solubilizers, solution accelerators, salt-forming agents, viscosity- and consistency-influencing agents, gel-forming agents, emulsifiers, solubilizers, wetting agents, spreading agents, antioxidants, preservatives, fillers and carriers, osmolarity regulators and mixtures thereof.

16. Use according to one of claims 1 to 15, **characterized in that** the phosphate-free pharmaceutical composition is present in the form of a solution, of drops, of a spray, of a suspension or emulsion, of a gel, of an ointment or paste, of a powder or granules, or of a tablet.

17. Use according to one of claims 1 to 16, **characterized in that** the phosphate-free pharmaceutical composition contains no moxaverin, heparin, panthenol and/or pantothenic acid.

18. Use according to one of claims 1 to 17, **characterized in that** the phosphate-free pharmaceutical composition contains no further pharmaceutical.

19. Use according to one of claims 11 to 18, **characterized in that** the phosphate-free pharmaceutical composition consists of at least one calcium chelating agent and at least one ophthalmologically compatible viscosity regulator and optionally a pharmaceutical excipient or a number of pharmaceutical excipients.

## Revendications

1. Utilisation d'au moins un chélateur de calcium et d'au moins un régulateur de viscosité acceptable sur le plan ophtalmologique à la preparation d'une composition pharmaceutique exempte de phosphates et d'agent conservateur pour le traitement et/ou la prévention de défauts de l'épithélium dans la cornée et/ou dans la conjonctive de l'oeil.

2. Utilisation selon la revendication 1, **caractérisée en ce que** les défauts de l'épithélium sont choisis parmi le groupe consistant en les défauts de l'épithélium causés par des effets mécaniques et/ou chimiques, des défauts de l'épithélium faisant suite à des interventions chirurgiacales, des défauts de l'épithélium par perturbation du mouillage de la surface de l'oeil, des défauts de l'épithélium par traitement de longue durée avec des compositions pharmaceutiques contenant des agents de conversation ou avec des lentilles de contact.

3. Utilisation selon la revendication 1 ou 2, **caractérisée en ce que** la composition pharmaceutique sans phosphates est utilisée pour une application de longue durée.

4. Utilisation selon la revendication 3, **caractérisée en ce que** l'application de longue durée est effectuée en cas de glaucome et en cas d'allergie.

5. Utilisation selon l'une quelconque des revendications 1 à 4, **caractérisée en ce que** des inclusions et/ou des dépôts difficilement solubles de composés de calcium dans la cornée et/ou dans la conjonctive, en particulier une calcification, sont empêchés et/ou sont réduits.

6. Utilisation selon l'une quelconque des revendications 2à 5, **caractérisée en ce que** les troubles du mouillage sont choisis parmi le groupe consistant en le syndrome de Sjögren, le syndrome xérophtalmique, et les perturbations du mouillage de l'oeil chez les porteurs de lentilles de contact.

7. Utilisation selon l'une quelconque des revendications 2 à 6, **caractérisée en ce que** les interventions chirurgicales sont choisis parmi le groupe consistant en les interventions chirurgicales sur la partie avant l'oeil, l'extraction de la cataracte, l'implantation d'une lentille, les interventions chirurgicales sur la réfraction, les interventions sur la cornée et les greffes de cornée, et les interventions sur la conjonctive.

8. Utilisation selon l'une quelqonque des revendications 1 à 7, **caractérisée en ce que** la composition pharmaceutique sans phosphates est exempte d'ions calcium.

9. Utilisation selon l'une quelqonque des revendications 1 à 8, **caractérisée en ce que** la quantité de régulateur de viscosité est d'environ 0,005% en poids à environ 5% en poids, de préférence d'environ 0,01% en poids à environ 1% en poids, chaque fois rapportés au poids total de la composition pharmaceutique sans phosphates.

10. Utilisation selon l'une quelqonque des revendications 1 à 9 **caractérisée en ce que** le régulateur de viscosité est choisi parmi le groupe consistant en le sulfate de chondroïtine, le polyacrylamide, l'acide polyacrylique, des résines polyacryliques, le polyéthylène glycol, des dérivés de la cellulose, des polysaccharides, l'alcool polyvinylique, la polyvinyle pyrrolidone, l'acide hyaluronique, les hyaluronates, des dérivés de ces derniers et leurs mélanges.

11. Utilisation selon la revendication 10, **caractérisée en ce que** l'acide hyaluronique, le hyaluronate et/ou leurs dérivés présentent un poids moléculaire qui est compris dans l'intervalle d'environ 50.000 à 10.000.000 daltons, de préférence d'environ 250.000 à environ 5.000.000 daltons.

12. Utilisation selon la revendication 10 ou 11, **caractérisée en ce que** le hyaluronate est le hyaluronate de sodium.

13. Utilisation selon l'une quelconque des revendications 1 à 12, **caractérisée en ce que** le chélateur de calcium est choisi parmi le groupe consistant en les citrates, l'acide critique, l'EDTA, l'EGTA, et des mélanges de ceux-ci.

14. Utilisation selon l'une quelconque des revendications là 13, **caractérisée en ce que** la composition pharmaceutique contient d'autres substances pharmaceutiques auxiliaires.

15. Utilisation selon la revendication 14, **caractérisée en ce que** les substances pharmaceutiques auxiliaires sont choisies parmi le groupe consistant en des substances tampons inorganiques, des substances tampons organiques, des sels inorganiques, des sels organiques, des régulateurs de viscosité, des solvants, des promoteurs de dissolution, des accélérateurs de dissolution, des halogèns, des substances que influent sur la viscosité et la consistence, des gélifiants, des émulsifiants, des solubilisateurs, des tensioactifs, des dispersants, des antioxydants, des agents de conversation, des substances de charge et de support, des régulateurs d'osmolarité ainsi que des mélanges de ceux-ci.

16. Utilisation selon l'une quelconque des revendications 1 à 15, **caractérisée en ce que** la composition pharmaceutique sans phosphates se présente sous la forme d'une solution, de gouttes, d'un spray, d'une suspension, d'une émuslion, d'un gel, d'une pommade, d'une pâte, d'une poudre, d'un granulé ou d'une tablette.

17. Utilisation selon l'une quelconque des revendications 1 à 16, **caractérisée en ce que** la composition pharmaceutique sans phosphates ne contient pas de moxavérine, d'héparine, de panthénol, et/ou d'acide pantothénique.

18. Utilisation selon l'une quelconque des revendications 1 à 17, **caractérisée en ce que** la composition pharmaceutique sans phosphates ne contient pas d'autre substance médicamenteuse.

19. Utilisation selon l'une quelconque des revendications 11 à 18, **caractérisée en ce que** la composition pharmaceutique sans phosphates consiste en au moins un chélateur de calcium et au moins un régulateur de viscosité acceptable sur le plan ophtalmologique, ainsi qu'en option en une substance pharmaceutique auxiliaire ou en plusieurs substances pharmaceutiques auxiliaires.
